# EUROPÄISCHE PATENTSCHRIFT

(11) **EP 1 261 300 B1**
(45) Veröffentlichungstag und Bekanntmachung des Hinweises auf die Patenterteilung: **16.06.2004**
(21) Anmeldenummer: 01919123.8
(22) Anmeldetag: 31.01.2001
(51) Int. Cl.: A61F 2/16

(54) **INTRAOCULARE LINSE**
INTRAOCULAR LENS
LENTILLE INTRAOCULAIRE

(30) Priorität: 04.03.2000 DE 10010683
(43) Veröffentlichungstag der Anmeldung: 04.12.2002
(73) Patentinhaber: Morcher GmbH, 70374 Stuttgart (DE)
(72) Erfinder: MORCHER, Olaf, Kurt, 70374 Stuttgart (DE)
(74) Vertreter: Rotermund, Hanns-Jörg, Dipl.-Phys.
(86) Internationale Anmeldenummer: PCT/DE2001/000402
(87) Internationale Veröffentlichungsnummer: WO 2001/066040

(56) Entgegenhaltungen:
- WO-A-00/67677
- DE-A- 3 926 536
- FR-A- 2 696 340
- RU-C- 2 106 832
- US-A- 4 955 904
- US-A- 5 662 706

## Beschreibung

Die Erfindung betrifft eine intraoculare Linse mit durchsichtigem Zentralbereich sowie daran radial anschließendem Iris-Diaphragma, geeignet zur Korrektur bzw. Abdeckung einer Aniridie, wobei Linse und Diaphragma als einstückige Einheit ausgebildet sind und das Diaphragma im wesentlichen aus einer flexiblen, opak pigmentierten Folie besteht, welche in einen an den Zentralbereich der Linse einstückig anschließenden Linsenaußenbereich eingebettet und vom transparenten Linsenmaterial vollständig umhüllt ist

Verletzungen eines Auges führen oftmals auch zu Verletzungen der Iris, so daß ein vergleichsweise hoher Bedarf für intraoculare Linsen mit Iris-Diaphragma besteht.

Auf dem Markt erhältliche derartige Linsen besitzen einen zweiteiligen Aufbau mit einem ähnlich einer Ringscheibe ausgebildeten Diaphragma aus schwarzem Polymethyl-Methacrylat. In der Öffnung des Diaphragmas ist eine Linse aus transparentem Polymethyl-Methacrylat mechanisch gehalten bzw. fixiert.

Diese Linsen haben sich in der Praxis bewährt.

Da diese Linsen jedoch vergleichsweise wenig flexibel sind, müssen zur Implantation der Linsen im Auge relativ große Operationsöffnungen ausgeführt werden, so daß eine relativ lange Zeit für die Heilung des Auges in Kauf genommen werden muß.

Des weiteren ist es erwünscht, das optische Erscheinungsbild des Diaphragmas der implantierten Linse noch stärker einer natürlichen Iris anzunähern. Allerdings ist es außerordentlich kostenaufwendig, für das Iris-Diaphragma bei diesen bekannten Linsen unterschiedlich gefärbte Materialien einzusetzen. Wenn statt dessen auf das schwarze Iris-Diaphragma Farbe aufgetragen wird, beispielsweise durch Bemalung oder Bedruckung, ergeben sich toxikologische Gefahren oder Probleme hinsichtlich der Langzeitstabilität.

Aus der US 4 955 904 sowie der US 5 662 706 sind maskierte intraoculare Linsen der eingangs angegebenen Art bekannt, wobei die Maskierung zur Korrektur von Abbildungsfehlern des Auges, nicht zum Ersatz einer Iris, dient. Gemäß der US 4 955 904 können zur Ausbildung der Maskierung dienende Farbstoffe in die Linse eingebettet sein.

Aufgabe der Erfindung ist es, eine verbesserte Linse zur Versorgung einer Aniridie zu schaffen.

Diese Aufgabe wird erfindungsgemäß dadurch gelöst, daß die Folie mehrschichtig ausgebildet ist und eine innere Pigmentträgerschicht sowie eine die Pigmentträgerschicht vollständig - auch an den Schichträndern - versiegelnde bzw. umhüllende und gegen Diffusion der Pigmente resistente Sperrschicht aufweist.

Die Erfindung beruht auf dem allgemeinen Gedanken, als Diaphragma ein vollständig mit dem transparenten Linsenmaterial umhülltes Folienteil einzusetzen, so daß einerseits der durchsichtige Zentralbereich der Linse einstückig in die Umhüllung der Folie übergehen kann und andererseits die Folie sowie die Pigmente durch das Linsenmaterial gegen eine Wechselwirkung mit dem biologischen Milieu um Auge abgeschirmt sind.

Gemäß der Erfindung soll die Folie mehrschichtig ausgebildet sein und eine innere Pigmentträgerschicht, welche sich vorzugsweise drucktechnisch farbig gestalten läßt, sowie eine die Pigmentträgerschicht vollständig - auch an den Schichträndern - versiegelnde bzw. umhüllenden und gegen Diffusion der Pigmente resistente Sperrschicht aufweisen.

Bei dieser Ausführungsform dient das die mehrschichtige Folie umhüllende transparente Linsenmaterial in erster Linie nur dazu, eine gute biologische Verträglichkeit der Linse im Auge zu gewährleisten, während die Sperrschicht auf der Folie die Versiegelung der Pigmente übernimmt.

Außerdem wird damit der besondere Vorteil geboten, daß als transparentes Linsenmaterial ohne weiteres die für faltbare Linsen üblichen Wasser aufnehmenden hydrophilen Materialien einsetzbar sind, so daß erstmals eine faltbare intraoculare Aniridie-Linse herstellbar ist.

Vorzugsweise wird als transparentes Linsenmaterial ein Copolymer aus Methyl-Methacrylat und 2-Hydroxyethyl Methacrylat eingesetzt. Dieses Material optimiert die Faltbarkeit und die Wasserretention der Linse während der chirurgischen Implantation im Auge und sichert zugleich die Langzeitstabilität im biologischen Milieu sowie die optische Qualität. Außerdem ist dieses Material hitzebeständig bzw. für eine Sterilisation mit Dampf geeignet.

Insbesondere wenn das Linsenmaterial ausgeprägt hydrophil ist und Wasser aufnimmt, sollte die Sperrschicht vorzugsweise aus einem hydrophoben Material bestehen, z.B. aus Silikon. Damit kann eine Diffusion von Pigmenten sicher verhindert werden. Darüber hinaus bleibt Silikon auch bei der Dampfsterilisation der Linse stabil.

Die Pigmentträgerfolie ist vorzugsweise aus einem hitzebeständigen, für die wünschenswerte Dampfsterilisation der Linse geeigneten Material, wie insbesondere Polypropylen und/oder Polimid, hergestellt und mit einem einer natürlichen Iris entsprechenden farbigen Muster bedruckt, z.B. durch Siebdruck.

Im übrigen wird hinsichtlich bevorzugter Merkmale der Erfindung auf die Ansprüche sowie die nachfolgende Erläuterung der Zeichnung verwiesen, anhand der besonders bevorzugte Ausführungsformen der erfindungsgemäßen Linse und deren Herstellung näher beschrieben wird.

Dabei zeigt
- Fig. 1: eine Draufsicht auf die erfindungsgemäße Linse,
- Fig. 2: ein Schnittbild entsprechend der Schnittlinie II-II in Fig. 1 und
- Fig. 3: ein der Fig. 2 entsprechendes Schnittbild einer abgewandelten Ausführungsform.

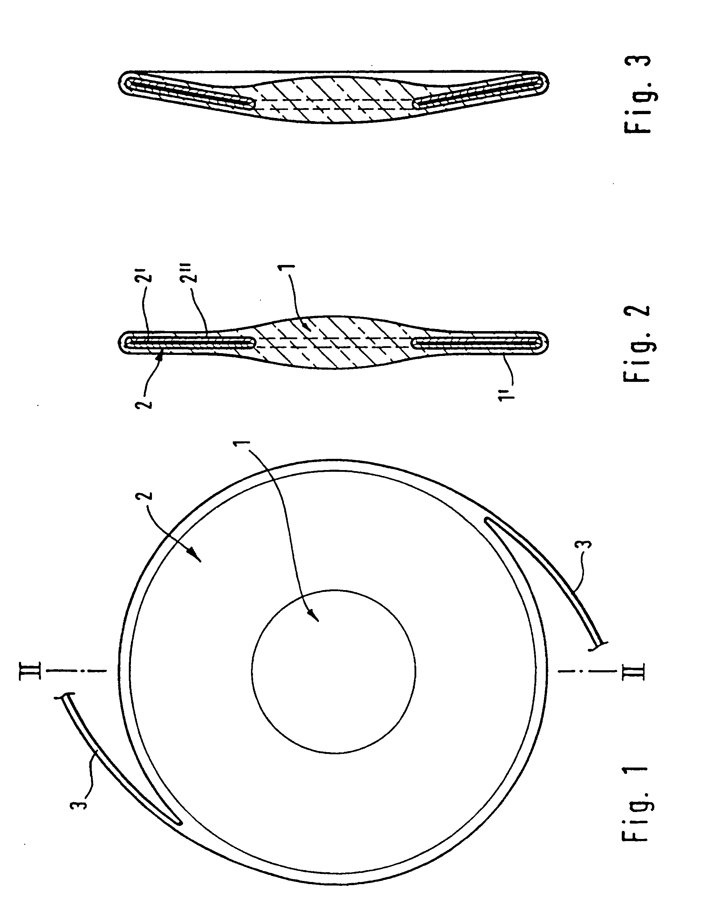

Die in den Fig. 1 und 2 dargestellte intraoculare Linse besitzt einen linsenförmigen, durchsichtigen Zentralbereich 1, an den sich nach radial außen ein undurchsichtiges, im wesentlichen ringscheibenförmiges Iris-Diaphragma 2 anschließt. Dieses besteht im wesentlichen aus einer ringscheibenförmigen dünnen Folie 2', die durch aufgedruckte Pigmente nach Art einer Iris gestaltet und praktisch undurchsichtig ausgebildet ist. Diese irisartige Pigmentträgerfolie 2' ist umhüllt mit einer Sperrschicht 2", welche auch an den Rändern der Pigmentträgerfolie 2' geschlossen ist.

Das gesamte Iris-Diaphragma 2 ist eingebettet in das transparente Material des Zentralbereiches 1, d.h. der Zentralbereich 1 setzt sich nach radial außen in eine das Iris-Diaphragma 2 einbettende Umhüllung 1' fort, welche mit ihrem Außenrand den Außenrand des Iris-Diaphragmas 2 umschließt.

Im übrigen können am Außenrand der Umhüllung 1' Haptikelemente 3 angeformt sein, welche einstückig in die Umhüllung 1' übergehen und aus dem gleichen transparenten Material bestehen.

Die Pigmentträgerfolie 2' besteht vorzugsweise aus Polypropylen und/oder Polimid. Die Versieglung 2" besteht zweckmäßigerweise aus Silikon, und der Zentralbereich 1 sowie die Umhüllung 1' sind bevorzugt aus einem Copolymer aus Methyl-Methacrylat und 2-Hydroxymethyl-Methacrylat gefertigt.

Die Herstellung der Linse der Fig. 1 und 2 kann dadurch erfolgen, daß zunächst die Pigmentträgerfolie 2' entsprechend dem Bild einer natürlichen Iris mit Pigmenten bedruckt und ringscheibenförmig, entsprechend einer Iris, ausgestanzt wird.

Sodann wird diese fertige Pigmentträgerfolie 2' mit der Sperrschicht 2" versiegelt.

Das auf diese Weise erzeugte Iris-Diaphragma 2 wird mit einem Copolymer aus Methyl-Methacrylat und 2-Hydroxymethyl-Methacrylat umgossen, so daß nach Aushärtung des Gußmaterials ein dicker Rohling vorliegt, der axial und radial über das Iris-Diaphragma 2 deutlich hinausragt.

Nachfolgend wird durch spanabhebende Bearbeitung des Rohlings die in den Fig. 1 und 2 dargestellte Linse mit den Haptikelementen 3 hergestellt.

Die fertige Linse wird dann einer Dampf-Sterilisation unterzogen und in ein steriles Salzwasserbad eingelegt. Auf diese Weise wird eine wasserhaltige, sehr flexible faltbare Linse geschaffen, welche durch kleine Operationsöffnungen im Auge implantiert werden kann.

Die Fig. 3 zeigt beispielhaft, daß die Linse abweichend von der Darstellung der Fig. 2 auch eine gewölbte Form aufweisen kann.

Im übrigen ist die bikonvexe Ausbildung des Zentralbereiches 1 der Linsen der Fig. 2 und 3 nur beispielhaft. Bei Bedarf ist auch eine plankonvexe oder konvex-konkave Ausbildung möglich.

Im übrigen kann der Außenrand der Umhüllung 1' wulstförmig ausgebildet sein, mit prinzipiell beliebigen Wulstquerschnitten, soweit dies medizinisch erwünscht ist.

Auch die dargestellte Form der Haptikelemente 3 ist lediglich beispielhaft. Andere Formen sind, gegebenenfalls nach ärztlichen Vorgaben, ohne weiteres möglich. Beispielsweise könnten die Haptikelemente 3 auch die Form von Ösen haben.

## Patentansprüche

1. Intraoculare Linse mit durchsichtigem Zentralbereich (1) sowie daran radial nach außen anschließendem Iris-Diaphragma (2), geeignet zur Korrektur bzw. Abdeckung einer Aniridie,
wobei Linse und Diaphragma (2) als einstückige Einheit ausgebildet sind und das Diaphragma im wesentlichen aus einer flexiblen, opak pigmentierten Folie (2', 2") besteht, welche in einen an den Zentralbereich (1) der Linse einstückig anschließenden Linsenaußenbereich (1') eingebettet und vom transparenten Linsenmaterial vollständig umhüllt ist,
**dadurch gekennzeichnet,**
**daß** die Folie mehrschichtig ausgebildet ist und eine innere Pigmentträgerschicht (2') sowie eine die Pigmentträgerschicht vollständig - auch an den Schichträndern - versiegelnde bzw. umhüllende und gegen Diffusion der Pigmente resistente Sperrschicht (2") aufweist.

2. Linse nach Anspruch 1,
**dadurch gekennzeichnet,**
**daß** das transparente Linsenmaterial hydrophil und das Material der Sperrschicht (2") hydrophob ist.

3. Linse nach einem der Ansprüche 1 oder 2,
**dadurch gekennzeichnet,**
**daß** die Pigmentträgerschicht aus einer farbig bedruckten Folie (2') besteht.

4. Linse nach einem der Ansprüche 1 bis 3,
**dadurch gekennzeichnet,**
**daß** der Zentralbereich (1) der Linse relativ zur Ebene des Iris-Diaphragmas (2) axial versetzt angeordnet ist.

5. Linse nach einem der Ansprüche 1 bis 4,
**dadurch gekennzeichnet,**
**daß** das transparente Linsenmaterial ein Copolymer aus Methyl-Methacrylat und 2-Hydroxyethyl-Methacrylat ist.

6. Linse nach einem der Ansprüche 1 bis 5,
**dadurch gekennzeichnet,**
**daß** die Folie (2') aus Polypropylen und/oder Polimid besteht.

7. Linse nach einem der Ansprüche 1 bis 6,
**dadurch gekennzeichnet,**
**daß** die Sperrschicht (2") aus Silikon besteht.

8. Verfahren zur Herstellung einer Linse nach einem der Ansprüche 1 bis 7, wobei zur Herstellung eines Iris-Diaphragmas (2) eine entsprechend einer natürlichen Iris mit Pigmenten bedruckte Folie (2') ausgestanzt und mit transparentem Linsenmaterial umgossen wird, und wobei ein nach Aushärtung des Linsenmaterials erhaltener Rohling spanabhebend entsprechend einer gewünschten Linsenform bearbeitet wird,
**dadurch gekennzeichnet,**
**daß** die ausgestanzte Folie (2') zunächst mit einer auch die Folienränder umhüllenden und gegen Diffusion der Pigmente resistenten Versiegelung (2") versehen wird.

9. Verfahren nach Anspruch 8,
**dadurch gekennzeichnet,**
**daß** eine zur Herstellung des Iris-Diaphragmas (2) dienende Folie (2') durch Siebdruck pigmentiert wird.

## Claims

1. An intraocular lens having a transparent central area (1) as well as an iris diaphragm (2) connected thereto on the outside radially to correct or cover an aniridia, wherein the lens and diaphragm (2) are designed as a one-piece unit and the diaphragm consists essentially of a flexible, opaque, pigmented film (2', 2") which is embedded in a lens outer area (1') that is connected in one piece to the central area (1) of the lens and is completely sheathed by the transparent lens material,
**characterized in that**
the film is formed as a multi-layer and has an inner pigment carrier layer (2') and a barrier layer (2") which completely sheaths and seals the pigment carrier layer - even at the edges of the layer - and is resistant to diffusion of the pigments.

2. The lens according to Claim 1,
**characterized in that**
the transparent lens material is hydrophilic and the material of the barrier layer (2") is hydrophobic.

3. The lens according to Claim 1 or 2,
**characterized in that**
the pigment carrier layer is made of a film (2') which is printed in color.

4. The lens according to one of Claims 1 through 3,
**characterized in that**
the central area (1) of the lens is arranged with an axial offset relative to the plane of the iris diaphragm (2).

5. The lens according to one of Claims 1 through 4,
**characterized in that**
the transparent lens material is a copolymer of methyl methacrylate and 2-hydroxymethyl methacrylate.

6. The lens according to one of Claims 1 through 5,
**characterized in that**
the film (2') is made of polypropylene and/or polyimide.

7. The lens according to one of Claims 1 through 6,
**characterized in that**
the barrier layer (2") is made of silicone.

8. A method of producing a lens according to one of Claims 1 through 7, wherein, for producing an iris diaphragm (2), a film (2') printed with pigments according to a natural iris is punched out and sealed (2") in transparent lens material, and wherein a blank obtained after curing the lens material is machined according to the desired lens shape,
**characterized in that**
the punched out film (2') is first provided with a sealing (2") also covering the film edges and being resistant to diffusion of the pigments.

9. The method according to Claim 8,
**characterized in that**
a film (2') which is used to produce the iris diaphragm (2) is pigmented by screen printing.

## Revendications

1. Lentille intraoculaire comprenant une zone centrale transparente (1) ainsi qu'un diaphragme iris (2) qui s'y raccorde radialement vers l'extérieur, adaptée à la correction ou à la couverture d'une aniridie, la lentille et le diaphragme (2) étant réalisés sous forme d'unité monobloc et le diaphragme se composant essentiellement d'une feuille flexible (2', 2") de pigmentation opaque, enrobée dans une zone extérieure (1') de la lentille se raccordant d'une seule pièce à la zone centrale (1) de cette dernière et totalement enveloppée par le matériau transparent de la lentille, **caractérisée en ce que** la feuille a une réalisation multicouche et présente une couche de substrat pigmentaire (2') intérieure ainsi qu'un couche de barrage (2") résistante à la diffusion des pigments, qui scelle ou enveloppe totalement la couche de substrat pigmentaire, également sur les bords de cette dernière.

2. Lentille suivant la revendication 1, **caractérisée en ce que** le matériau transparent de la lentille est hydrophile et le matériau de la couche de barrage (2") hydrophobe.

3. Lentille suivant l'une des revendications 1 et 2, **caractérisée en ce que** la couche de substrat pigmentaire se compose d'une feuille (2') imprimée en couleur.

4. Lentille suivant l'une des revendications 1 à 3, **caractérisée en ce que** la zone centrale (1) de la lentille est disposée en déport axial par rapport au plan du diaphragme iris (2).

5. Lentille suivant l'une des revendications 1 à 4, **caractérisée en ce que** le matériau transparent de la lentille est un copolymère de méthacrylate de méthyle et de 2-hydroxyéthyl-méthacrylate.

6. Lentille suivant l'une des revendications 1 à 5, **caractérisée en ce que** la feuille (2') se compose de polypropylène et/ou de polimide.

7. Lentille suivant l'une des revendications 1 à 6, **caractérisée en ce que** la couche de barrage (2") se compose de silicone.

8. Procédé de fabrication d'une lentille suivant l'une des revendications 1 à 7, une feuille (2') imprimée de pigments, en conformité avec un iris naturel, étant découpée pour la fabrication d'un diaphragme iris (2) et coulée dans un matériau de lentille transparent, et une ébauche obtenue après le durcissement du matériau de la lentille étant usinée en fonction d'une forme de lentille souhaitée, **caractérisé en ce que** la feuille découpée (2') est d'abord munie d'un scellage (2") résistant à la diffusion des pigments et enveloppant également les bords de la feuille.

9. Procédé suivant la revendication 8, **caractérisé en ce qu'**une feuille (2'), servant à la fabrication du diaphragme iris (2), est pigmentée par sérigraphie.
